# EUROPEAN PATENT SPECIFICATION

(11) **EP 2 533 828 B1**
(45) Date of publication and mention of the grant of the patent: **27.02.2019**
(21) Application number: 11705110.2
(22) Date of filing: 09.02.2011
(51) Int. Cl.: A61M 1/36, A61M 1/38, A61K 31/727, A61P 31/00

(54) **REMOVAL OF VIRULENCE FACTORS THROUGH EXTRACORPOREAL THERAPY**
ENTFERNUNG VON VIRULENZFAKTOREN DURCH EXTRAKORPORALE THERAPIE
ÉLIMINATION DE FACTEURS DE VIRULENCE PAR THÉRAPIE EXTRACORPORELLE

(30) Priority: 09.02.2010 US 302826 P
(43) Date of publication of application: 19.12.2012
(73) Proprietor: ExThera Medical Corporation, Martinez, CA 94553 (US)
(72) Inventor: MCCREA, Keith, Concord, California 94518 (US); WARD, Robert S., Orinda, CA 94563 (US)
(74) Representative: AWA Sweden AB
(86) International application number: PCT/US2011/024229
(87) International publication number: WO 2011/100354

(56) References cited:
- EP-A2- 1 044 696
- WO-A2-2008/157570
- US-A1- 2009 136 586
- THOMAS RICHARD ET AL: "Common oligosaccharide moieties inhibit the adherence of typical and atypical respiratory pathogens.", JOURNAL OF MEDICAL MICROBIOLOGY SEP 2004 LNKD- PUBMED:15314189, vol. 53, no. Pt 9, September 2004 (2004-09), pages 833-840, XP002637452, ISSN: 0022-2615
- DIXON T C ET AL: "ANTHRAX", NEW ENGLAND JOURNAL OF MEDICINE, THE, MASSACHUSETTS MEDICAL SOCIETY, WALTHAM, MA, US, vol. 341, no. 11, 9 September 1999 (1999-09-09), pages 815-826, XP001042176, ISSN: 0028-4793, DOI: DOI:10.1056/NEJM199909093411107
- SWARTZ MORTON N: "Recognition and management of anthrax: An update", NEW ENGLAND JOURNAL OF MEDICINE, vol. 345, no. 22, 29 November 2001 (2001-11-29), pages 1621-1626, XP002637453, ISSN: 0028-4793

## Description

### FIELD OF THE INVENTION

The present invention is directed to a method for removing pathogens and/or toxins released from pathogens from blood or serum (blood) by contacting the blood with a solid, essentially nonporous substrate which has been surface treated with heparin, heparin sulfate and/or other molecules or chemical groups (the adsorbent media or media) having a binding affinity for the pathogens and/or toxins to be removed (the adsorbents). The invention can be used to remove virulence factors, e.g. toxins, that are released from pathogens such as *Bacillus anthracis, Pseudomonas aeruginosa,* and *Staphylococcus aureus.* In one aspect, the size of the interstitial channels within said media is balanced with the amount of media surface area and the surface concentration of binding sites on the media to provide adequate adsorptive capacity while also allowing relatively high flow rates of blood past the media.

There is also described a method of treating a disease by removing pathogens and/or toxins from the pathogens from blood by contacting blood with an essentially nonporous substrate coated with heparin and/or other adsorbent materials, and a device for performing the method and treatment.

### BACKGROUND

Various disease conditions are characterized by the presence of elevated concentrations of pathogens and/or toxins in the blood stream. Some such conditions are treated by therapies designed to kill the pathogen, e.g. through the administration of anti-infective pharmaceuticals. Some other conditions are treated by therapies that attempt to reduce the concentration of blood-borne pathogens or toxins in the patient. Other diseases are treated by therapies that attempt to directly remove only specific components from the patient's blood.

For example, Guillian-Barre syndrome is currently understood to be an autoimmune disorder triggered by viral infection that stimulates the body's immune system to over produce antibodies or other proteins which can attack the patient's nervous system, causing increasing levels of paralysis. Most patients recover over time, though such patients appear to be susceptible to recurrence of the condition from subsequent viral infections. One method for treating Guillian-Barre syndrome involves plasmapheresis to 'clean' the patient's blood by removing antibodies believed to be attacking the patient's nervous system.

Heparin is a polysaccharide, that can be isolated from mammalian tissue. It has a very specific distribution in mammalian tissue; being present only in the basophilic granules of mast cells. Since its discovery in 1916 by the American scientist McLean, heparin has been recognized for its ability to prevent blood from clotting, and for its relatively short half-life in the body. Systemic heparin, administered by injection of the free drug, has been used clinically for more than 50 years as a safe and effective blood anticoagulant and antithrombotic agent. The effects of heparin on blood coagulation/clotting diminish fairly quickly after administration is halted, making its use during surgery and other procedures effective and safe. That is, heparin's anticoagulant and antithrombogenic properties are useful during many medical procedures, for example to minimize undesirable interactions between blood and the man-made surfaces of extracorporeal circuits. Once the procedure is over, the administration of heparin may be then terminated. The heparin concentration in the patient's blood diminishes to a safe level within a few hours. This is particularly important following surgery when healing depends on the ability of blood to clot at the surgical site to avoid bleeding complications. In addition to its well established and continuing use in the treatment of thromboembolic disorders and the prevention of surface-induced thrombogenisis, heparin has more recently been found to have a wide range of other functions apparently unrelated to its function as an anticoagulant. For example, a large number of proteins in blood are now known to bind with high affinity, to heparin and/or the closely-related polysaccharide heparin sulfate which is also found in animal tissue, including the luminal surface of healthy blood vessels. Examples are antithrombin (AT), fibronectin, vitronectin, growth factors (e.g. the fibroblast growth factors, the insulin like growth factors, etc.). Human serum albumin (HSA) also binds, but with a lower affinity despite its high concentration in blood.

Utilizing the selective adsorption properties of systemic/free heparin for hindering infections, by introducing heparin fragments and/or so-called sialic-containing fragments into the vascular system has previously been considered. This proposed therapy was based on the assumption that these fragments would bind to the lectins on the microbes and block them so they could not bind to the receptors on the mammalian cell surface. Although this approach has been investigated by many scientists, only limited success has been reported to date. The most common problem has been bleeding complications associated with the large amounts of free heparin introduced into the blood stream to achieve a clinically-useful reduction of pathogenic microbes. The present invention does not require the use of free *systemic* heparin and thus avoids bleeding complications. This is accomplished by permanently binding the heparin or heparin sulphate to a solid substrate with high surface area, and exposing the blood to a cartridge or filter containing this adsorption media.

One particular disease of importance to treat is anthrax. The bacterium *Bacillus anthracis* is a naturally occurring bacterium that produces spores that can remain dormant for years, e.g. in the soil or on animals. The disease can be fatal to animals. For human infections, the spores have to enter the body, usually through a cut in the skin or by consuming contaminated meat. But recently, concern about bioterrorism has focused attention on infections caused by inhaling the spores. When inhaled, the body's immune system can quickly become overwhelmed and go into shock.

Anthrax toxin (also called "anthrax lethal toxin", or "LT") consists of three nontoxic proteins that associate in binary or ternary combinations to form toxic complexes at the surface of mammalian cells. One of these proteins, protective antigen (PA), transports the other two, edema factor (EF) and lethal factor (LF), to the cytosol. LF is a Zn2+-protease that cleaves certain MAP kinase kinases, leading to death of the host via a poorly defined sequence of events. EF, a calmodulin- and Ca2+-dependent adenylate cyclase, is responsible for the edema seen in the disease. Both enzymes are believed to benefit the bacteria by inhibiting cells of the host's innate immune system. Assembly of toxic complexes begins after PA binds to cellular receptors and is cleaved into two fragments by furin proteases. The smaller fragment dissociates, allowing the receptor-bound fragment, PA63 (63 kDa), to self-associate and form a ring-shaped, heptameric pore precursor (prepore). The prepore binds up to three molecules of EF and/or LF, and the resulting complexes are endocytosed and trafficked to an acidic compartment. There, the prepore converts to a transmembrane pore, mediating translocation of EF and LF to the cytosol. Recent studies have revealed (a) the identity of receptors; (b) crystallographic structures of the three toxin proteins and the heptameric PA63 prepore; and (c) information about toxin assembly, entry, and action within the cytosol. Knowledge of the structure and mode of action of the toxin has unveiled potential applications in medicine, including approaches to treating anthrax infections. Collier, R. J., Rev. Microbiol., 2001 ;27(3):167-200.

Two human cellular receptors for PA have been identified. One is called anthrax toxin receptor (ATR) coded by the tumor endothelial marker 8 (TEM8) gene. It occurs more than ten thousend fold on the surface of macrophage cells lines. A truncated, soluble form of ATR (lacking the membrane anchoring sequence) is able to protect cell cultures against the lethal action of anthrax toxin. ATR is expressed in a variety of tissues including the central nervous system, heart, lung, and lymphocytes. The ATR cDNA codes for a Protein of 368 amino acids. It is predicted to have a 27 amino acid leader sequence, an extracellular domain of 293 amino acids, a 23 residues transmembrane region, and a short cytoplasmic tail at the carboxy terminal.

Another cellular protein with receptor function for PA is the capillary morphogenesis protein 2 (CMG2). Both ATR and CMG2 contain a domain structurally related to von Willdebrand factor type A (VWA), which is involved in binding. The structure of CMG2-VWA is known.

Like other bacilli *Bacillus antrhacis* is able to differentiate into dormant spores, which may last for years in spite of adverse environmental conditions. The spores will germinate to vegetative cells as soon as nutrients are available. This may occur on the skin or within the lung of a human or animal. Once inside a body the bacilli grow to high titers, aided by toxin they overcome host defense. Besides the toxin other components (a poly-D-glutamic acid capsule) contribute to virulence. Both capsule and toxins are coded on plasmids harboured by the bacteria.

Protection against infection may be gained by vaccination. Licensed vaccines are spores from toxigenic but nonencapsulated *B*. *anthracis* or aluminum hydroxide absorbed cell-free PA. The use of the attenuated live vaccines may have local adverse responses and are not very effective. A still experimental vaccine was constructed by engineering the PA gene into an originally plasmidless bacterial strain. Human vaccination is not usually done as natural anthrax infections are rather rare.

In early stages infections are cured by antibiotics, with ciprofloxacin as the drug of choice. Unrecognized infections usually are fatal. Anti-toxin treatment (e.g. with immunoglobulins directed against PA or synthetic peptides competing for binding of the toxin factors) may help to overcome a sever infection.

### BRIEF DESCRIPTION OF THE DRAWINGS

Figure 1. A and B) Exponential cultures of 7702 and 9131 were applied to control or heparin beads followed by ELISA-type assay to determine changes in PA amounts (reported as absorbance values) B) FBS was passed over both control and heparin beads 5 times prior to the addition of supernatents
Figure 2. Protection of macrophages from PA by heparinized media.
Figure 3. Reduction of USA300 MRSA α -toxin after passing over heparinized beads.

### SUMMARY OF THE INVENTION

An object of the present invention is to provide a method for the removal from mammalian blood of a toxin from a pathogen wherein said pathogen is a member selected from the group consisting of *Bacillus anthracis, Pseudomonas aeruginosa,* and *Staphylococcus aureus,* wherein the toxin from *Bacillus anthracis* is a member selected form the group of the anthrax lethal toxin, anthrax protective antigen, anthrax edema factor, anthrax lethal factor, anthrax polyglutamic acid capsule, anthralysin O, anthralysin B; wherein the toxin from *Pseudomonas aeruginosa* is Las A; and wherein the toxin from *Staphylococcus aureus* is a member selected from the group consisting of S. aureus α-toxin, and S. aureus β-toxin, said method comprising:
a. bringing a sample of blood in contact with a carbohydrate immobilized on a solid substrate, said carbohydrate having binding affinity for said toxin, under conditions allowing the binding of said substrate to said toxin in said sample of blood;
b. separating the sample from said substrate whereby said toxin is at least partially retained or said substrate and the removed sample has a reduced amount of toxin.

Another object of the invention is to provide an extracorporeal treatment of mammalian blood, in conjunction with a therapy for treating diseases caused by *Bacillus anthracis, Pseudomonas aeruginosa* or *Staphylococcus aureus* by removing the pathogens or toxins from the pathogens from mammalian blood by contacting mammalian blood with a solid essentially nonporous substrate coated with heparin (and optionally other adsorbent molecules) to thereby remove said pathogens or toxins.

### DETAILED DESCRIPTION OF THE INVENTION

### 1. Removal of Pathogens or Toxins from the Blood

A first aspect of the present invention provides a method for the removal of a toxin from blood, such as mammalian blood, by contacting the blood with a solid substrate e.g., coated with heparin.

In this method, heparin is immobilized onto the surface of the substrate. The inventors have found that immobilized heparin bound to a surface is effective for removing a significant amount of pathogens, toxins and/or virulence factors from blood. Virulence factors are toxins released from pathogens such as *B. anthracis, S. aureus,* and *P. aeruginosa* that allow them to colonize host cells, evade the host immune response, allow entry into and exit out of host cells, and obtain nutrition from cells. Many virulence factors target proteoglycans such as heparan sulfate on cell surface syndecans. Heparin is very similar in structure to heparan sulfate and will also bind virulence factors. Therefore, passing infected blood through a high-surface area cartridge with surface bound carbohydrates, such as heparin, can remove virulence factors that would normally compromise endothelial cell surfaces leading to colonization. By removing these virulence factors, the endothelial cell surface will be more protected and allow for traditional antibiotics to kill harmful bacteria causing the infection.

Syndecans are transmembrane proteins that contain heparan sulphate proteoglycan segments (HSPGs) and are present on most cell types. HSPGs have been known for some time to regulate a variety of biological processes, ranging from coagulation cascades, growth factor signaling, lipase binding and activity, cell adhesion to ECM and subsequent cytoskeletal organization, to infection of cells with microorganisms. They are complex molecules, with specific core protein to which a variable number of glycosaminoglycan (GAG) chains are attached. Not only the number of chains varies: although syndecans mainly bear heparan sulfate GAGs, some have additional chondroitin/dermatan sulfate chains. Furthermore, heparan sulfate chains can vary in length, epimerization of glucuronic acid to iduronic acid, overall sulfation of the chains, and in the position of sulfonation of the monosaccharides. Anne Woods, J. Clin, Invest. 2001;107(8):935-941.

The most common syndecan on endothelial cells is Synd1 and is responsible for binding and regulating a wide variety of molecules. These include growth factor and cytokines. Syndecan-1 was the first HSPG of this family to be identified and cloned. It is mainly limited to epithelial cells, but it is also found in condensing mesenchyme during development and in pre-B lymphocytes and plasma cells. Consistent with a role in adhesion, syndecan-1 present in a basolateral distribution in epithelia, and it appears to regulate epithelial morphology, since transfection of epithelial cells with antisense mRNA for syndecan-1 results in an epithelial-mesenchymal switch and activates cells to invade collagen gels. The attendant loss of E-cadherin in these cells suggests a coordinate regulation of syndecan-1 and E-cadherin, and indeed, reduced E-cadherin expression can also result in decreased syndecan-1 production.

Early studies indicated that syndecan-1 may be involved in cell adhesion to ECM. Transfection of syndecan-1 into Schwann cells, which normally lack this syndecan, increases spreading and promotes the formation of focal adhesions and stress fibers. Although syndecan-1 co-distributes with the microfilament system at the membrane during spreading and becomes detergent insoluble, it does not localize at focal adhesions. Indeed, there has been only one report of syndecan-1 being present in focal adhesions. Interestingly, detergent-insolubility, which is usually taken to indicate a linkage to the cytoskeleton, does not require the presence of the cytoplasmic domain; a very recent study indicates that syndecan-1 transmembrane domains associate with detergent-insoluble lipid rafts as part of a specialized type of endocytosis. Anne Woods, J. Clin, Invest. 2001;107(8):935-941.

Syndecans also aid in morphogenesis, host defense, and tissue repair. It is believed that sydecans can help prevent colonization and entry of bacteria and viruses into cells and that a potential mechanism of virulence by pathogens occurs when pathogens release virulence factors that bind to the heparin sulfate segments on the syndecans and then accelerates the shedding of the syndecans from the cell surface. This then allows bacterial cells and viruses to colonize the cell surface and enter the cell itself. This mechanism has been proposed for several pathogens, including *B*. *anthracis, S. aureasu,* and *P. aeruginosa* that are identified to subvert Synd1 during pathogenesis. These pathogens have been shown to accelerate Synd1 ectodomain shedding from epithelial cells, thereby comprising the epithelial barrier integrity. Popova, T. et al., BMC Microbiology, 2006, 6:8, 7 February 2006, "Acceleration of epithelial cell syndecan-1 shedding by anthrax hemolytic virulence factor". All three of the pathogens appear to target synd1. Chen Y. et al., Mol. Cells, 26, 415-426, Nov. 30, 2008, "Microbial Subversion of Heparan Sulfate Proteoglycans,".

In the present invention, a blood filtration/adsorption cartridge with a high-surface area of surface-bound heparin is employed, in which infected blood is passed over the surface of the surface-bound heparin. Excreted virulence factors in the blood will bind to the surface-bound heparin and can therefore be removed from the blood. By removing a large concentration of virulence factors from the blood, less colonization and damage to the endothelial cell surface will occur and allow for more time for conventional anti-microbial therapy to occur. In addition, when bacteria in the blood come in contact with the high-surface area heparin cartridge, the bacteria will release virulence factors which will bind to the surface bound heparin directly.

This therapeutic tool can then be used to treat serious infectious diseases such as *B*. *anthracis, S. aureus,* and *P. aeruginosa* blood infections. Treatment of infections by the present invention caused by any of the three pathogens can remove, partially or fully the following toxins from the blood:
a) for *B*. *anthracis*
   The Tripartite Protein toxin (Anthrax toxin) comprising
   Protective antigen (PA)
   Edema factor (EF)
   Lethal factor (LF)
   Polyclutamic acid capsule
   Anthralysin O (AnIO)
   Anthralysin B (AnIB)
   Lethal toxin (LT)
b) for *S*. *aureus*
   α-toxin
   β-toxin
c) for *P. aeruginosa*
   Las A

The flow rates typical of extracorporeal blood circuits require that the adsorbent 'bed' be designed to allow relatively high flow rates to operate safely.

In one aspect the present invention the substrate is designed with sufficiently large interstitial dimensions to permit a high flow rate of blood over the substrate without a large pressure drop. That is, as blood is taken from a mammalian patient, it is passed over the substrate at a flow rate whereby the delivery of adsorbates to the surface of the adsorbent bed is characterized primarily by forced convection. This is in contrast to the much slower process of molecular diffusion characteristic of highly porous media (e.g. porous silica, sephadex, crosslinked polystyrene size exclusion media,dense or microporous hollow-fiber or sheet membranes, etc.) used, for example, in size exclusion chromatograpy or other forms of affinity therapy.

This aspect of the invention provides sufficient adsorbtive capacity within the range of safe flow rates of > 50 mL/minute, which is in direct contrast to the much slower *diffusive* transport of adsorbates typical of many porous adsorbent media, which require adsorbates to diffuse through a microporous membrane, and/or into microscopic pores of the adsorbent media before binding to adsorption sites on or within the media, and which therefore require very low flow rates to achieve significant separations during each passage of blood.

The binding of toxins by heparin during convection transport is particularly effective under the relatively high-flow conditions typically employed in the (safe) operation of extracorporeal blood circuits, e.g. around > 50 mL/minute and preferably >150 mL/minute but less than about 2000 mL/minute. Adsorption within the pores of microporous media, in contrast, may require much lower flow rates through adsorption beds of practical size in order to achieve an adequate separation or purification, ie. < 50 mL/min to as low as < 1 mL/min.

It is recognized that, strictly speaking, it is 'residence time' on the adsorption column that needs to be much longer for a media requiring diffusive transport of adsorbates to the adsorbent site within the media and/or through a microporous membrane, when compared to the lower residence time needed to convey an adsorbate to the binding site (on an essentially nonporous media) by forced convection. However, there are practical limits to the dimensions of a safe and effective adsorbent cartridge, column, filter, etc., especially with respect to the maximum hold-up volume of blood it can contain, and the flow velocity of blood or serum past the adsorption media. For this reason average flow rate through the adsorption device is considered to be an important design variable.

Convection kinetics and diffusion kinetics can be compared in the removal of cytokines or pathogens from flowing blood: Adsorption media that depend on diffusion transport generally use very porous materials with extremely high internal surface area due to the presence of microscopic pores. Media suited for convection transport, on the other hand, generally rely on macroscopic "channels" or visible interstices between solid, essential nonporous material, such as particles, beads, fibers, reticulated foams, or spiral wound cartridges.

Media that rely on forced convection transport are generally more suitable for high-flow rates, while media that rely on the much slower diffusion transport are much less effective when high flow rates and shorter residence times are employed. For this reason, in an extracorporeal blood purification device, an adsorption media that does not require the adsorbate to slowly diffuse into pores within the adsorbent media is much preferred. When blood is pumped through circuits fabricated from man-made materials it is a general practice to employ relatively high blood flow rates in order to prevent stagnation and reduce the risk of clotting. On the other hand, *extremely* high flow rates must be avoided because they can expose blood cells to high shear rates and impingement damage that can rupture blood cells. The present invention, therefore, provides a method and device for removing cytokines or pathogens from blood using the preferred characteristics of convection transport and its desirable, more-rapid kinetics. This is achieved by passing/flowing blood over an essentially non-porous substrate that has been surface treated with adsorbent molecules, e.g. heparin, and which is therefore capable of binding the desired cytokine or pathogens to remove them from the blood.

The methods of the invention are intended to be applied in extracorporeal therapies or procedures, although implantable devices are also described. "Extracorporeal therapies" means procedures that are conducted outside the body, such as therapies in which desired products like oxygen, blood-anticoagulants, anesthetics etc can be added. Conversely, undesired products like naturally occurring toxins or poisons can be also removed from body fluids with specific types of extracorporeal circuits. Examples are haemodialysis and haemofiltration which represent technologies whereby blood is depleted of waste products. Adsorption on activated carbon has been used to remove blood-borne poisons, and so forth. Whole blood and blood serum from mammals can be used in the present invention. The amount of blood or blood serum that can be used in the claimed methods is not intended to be limited. It can range from less than 1 mL to above 1 L, up to and including the entire blood volume of the patient when continuous recirculation back to the patient is employed. One or more 'passes' through the adsorption bed may be used if needed. The blood may be human or animal blood.

Surface-heparinized adsorption media to remove pathogens or toxins from blood can be optimized according to the present invention for use in traditional extracorporeal blood circulation with flow rates > 50 mL/min, and preferably between about 150 and 2000 mL/min. Such high flow rates create short residence times within the adsorption column and convection transport dominates over Brownian diffusion transport. This is particularly important for binding large MW proteins or cytokines such as TNF-α and larger particles such as viruses, bacteria and parasites because they diffuse very, very slowly. In the present invention the dominant adsorption sites available for removing pathogens and toxins lie at the surfaces within the interstices of the media bed through which the blood flows or is delivered by forced convection. To treat blood, the interstitial channels need to be large enough to allow the transport of red blood cells, which are an average 6 microns in diameter. To allow a packed adsorption cartridge to be placed into an extracorporeal circuit with high blood flow rate, the interstitial channels must be several times larger than the diameter of red blood cells. This can prevent high shear rates that lead to hemolysis while simultaneously minimizing pressure drop in the blood that flows through the packed bed or cartridge. Additionally, the media is preferably rigid to minimize deformation that could clog the filter cartridge by compaction. Based on these preferences, an optimized rigid media balances interstitial channel size and total surface area, e.g., for efficient removal of cytokines in high-flow extracorporeal blood circuits.

### 2. The substrate used in the invention.

Various materials, in shape and composition, can be used as a substrate in the present invention. All suitable substrates provide high surface area while promoting the conveyance of adsorbates to the adsorbent sites that bind them (primarily) by forced convective transport. The media is typically provided packed within a container, such as a column, that is designed to hold the media so that it will not be carried away in the flowing blood (a.k.a. media migration) and permit the flow of blood past essentially all of the media's surface. Useful substrates for creating the adsorption media include non-porous rigid beads or particles, microparticles, reticulated foams, a rigid monolithic bed (e.g. formed from sintered beads or particles), a column packed with woven or non woven fabric, a column packed with a yarn or solid or hollow (but not microporous) monofilament fibers, a spiral wound cartridge formed from flat film or dense membrane, or a combination of media such as a mixed bead/fabric cartridge. A suitable substrate for use in the present invention may *initially* be microporous if it is rendered essentially non-porous during the surface modification process, for example.

In certain embodiments of the invention, the material of said solid substrate can be glass, cellulose, cellulose acetate, chitin, chitosan, crosslinked dextran, crosslinked agarose, polypropylene, polyethylene, polysulfone, polyacrylonitrile, silicone, Teflon or polyurethanes.

The surface concentration of the heparin on the solid substrate can be in the range of 1-10 µg/cm².

A column-type adsorption/filtration bed of the current invention has a macroporous structure that presents a high surface area to the blood or serum while preventing a large pressure drop and high shear rates. In addition to the potential for damaging the blood by hemolysis, high pressure drops should be avoided because they can shut down extracorporeal circuits equipped with automatic shut offs that respond to pressure drop.

### 2.1. Beads as Substrate

One useful substrate is in the form of solid beads or particles. The 'beads' can be made of materials that are sufficiently rigid to resist deformation/compaction under the encountered flow rates. Resistance to deformation is necessary to maintain the free volume and subsequent low pressure drop of the packed bed 'contactor'. The substantial lack of pores in the bulk of the substrate eliminates the need for adsorbates to diffuse into the pores prior to adsorption. The adsorption sites of the present invention are primarily on the surface of the media and are thus positioned to be accessible to adsorbates in the blood delivered to that surface largely by forced convection transport. Suitable substrates need not be perfectly smooth on their surface since roughness produces a desirable increase in surface area for attachment of binding sites, e.g. by covalent or ionic bonding of heparin.

Internal pores with molecular dimension, on the other hand, are largely avoided to eliminate the need for adsorbates to diffuse into the pores before attaching to binding sites.

Various kinds of beads can be used in the invention. Useful beads should have sufficient rigidity to avoid deformation/compaction during use in the method, and have sufficient surface area to be capable of being coated with heparin for use in the method.

Evidence of sufficient substrate rigidity is the absence of a significant increase in pressure drop across the adsorption bed during about one hour of flow of water or saline at rates typical of clinical use: for example, <10-50% increase relative to the initial pressure drop (measured within the first minute of flow) when measured at similar flow rate, e.g, of saline.

The beads or other high-surface-area substrates may be made of biocompatible materials, such as polymers or non-polymeric material, that is essentially free of leachable impurities including glass, cellulose, cellulose acetate, chitin, chitosan, crosslinked dextran, crosslinked alanese, polyurethane, polymethylmethacrylate, polyethylene or co-polymers of ethylene and other monomers, polyethylene imine, polypropylene, polysulfone, polyacrylonitrile, silicone and polyisobutylene. Examples of useful substrates include nonporous Ultra High Molecular Weight PolyEthylene (UHMWPE). Other suitable beads are polystyrene, high density and low density polyethylene, silica, polyurethane, and chitosan.

Methods for making such beads are *per se* known in the art. Polyethylene beads and other polyolefin beads are produced directly during the synthesis process and can often be used without further alteration.

As noted above, for use in the method of the invention, the size of the channels or interstitial space between individual beads for extracorporeal blood filtration should be optimized to prevent a high-pressure drop between the inlet and outlet of the cartridge, to permit safe passage of the blood cells between the individual beads in a high flow environment, and to provide appropriate interstitial surface area for binding of the heparin to the cytokines or pathogens in the blood. In a close packed bed of 300 micron beads, an appropriate interstitial pore size is approximately 68 microns in diameter. Useful beads have a size ranging from 100 to 500 microns in diameter. The average size of the beads can be from 150 to 450 microns. For example, polyethylene beads from DSM PTG, Berkeley, CA (formerly The Polymer Technology Group) having an average diameter of 0.3 mm are suitable. The interstitial pore is a function of bead size.

For use, the suitable beads are housed in a container, such as a column.

### 2.2. Other suitable forms of substrate

Reticulated foams have open cells and can be made from, for example, polyurethanes and polyethylenes. Control of pore size can be achieved through controlling the manufacturing method. In general, reticulated foams can have between 3 and 100 pores/inch and can exhibit a surface area of up to 66 cm²/cm³.

Beads can be sintered into a monolith porous structure through either chemical or physical means. Polyethylene beads can be sintered by heating the beads above their melting temperature in a cartridge and applying pressure. The resulting interstitial pore size is slightly reduced from the interstitial pore size of the packed bed of beads.

A column can be packed with either woven or non-woven heparinized fabric. By controlling the fiber size of fabric, the interstitial pore size can be controlled. Non-woven fabrics are also known as felts, and have a random orientation held together by entanglement of the fibers and adhesion. Woven fabrics have a defined non-random structure.

A column can be packed with fibers or yarns made from fibers. Polyethylene, and other fibers, can be drawn into thin hollow or solid fibers, that can be packed into cartridges similar to conventional hemodialysis cartridges. Additionally, these fibers can be woven into a yarn. Dyneema Purity® is a high strength woven fiber made of UHMWPE. Dyneema fiber can be heparinized and packed into a cartridge and provide a high-surface area support for the removal of cytokines and pathogens.

A spiral wound cartridge contains a thin membrane that is tightly wound together with optional spacer materials to prevent contact of adjacent surfaces. The membrane can be made from polymers such as polyurethane, polyethylene polypropylene, polysulfone, polycarbonate, PET, PBT, etc.

### 2.3. Attachment of heparin

The adsorption media of the present invention can comprise heparin covalently linked to the surface of the solid substrate. Various *per se* known methods can be used to attach heparin to the desired substrate, such as described in a review article by Wendel and Ziemer. (H.P Wendel and G. Ziemer, European Journal of Cardio-thoracic Surgery 16 (1999) 342-350). In one embodiment, the heparin is linked to the solid substrate by covalent end-point attachment. This method increases the safety of the device by reducing or eliminating the release of heparin from the substrate surface that could enter the blood stream. 'Leaching' of heparin by and into the blood is to be avoided because it can increase the risk of bleeding and heparin-induced thrombocytopenia.

Covalent attachment of heparin to a solid substrate provides better control of parameters such as surface density and orientation of the immobilized molecules as compared to non-covalent attachment. These parameters have been shown by the inventors to be important in order to provide optimal cytokine or pathogen binding to the immobilized carbohydrate molecules. The surface concentration of heparin on the solid substrate can be in the range of 1-10 µg/cm².Covalent end-point attachment means that heparin is covalently attached to the solid substrate via the terminal residue of the heparin molecule. Heparin can also be bound at multiple points. The end-point attachment is preferred.

If beads are used, they can be hydrophilized prior to attachment of the heparin or other compound. Possible methods of preparing the beads include acid etching, plasma treating, and exposure to strong oxidizers such as potassium permanganate.

### 2.4. Amount of heparin/gram substrate

The amount of heparin per gram substrate can vary. If beads are used, the amount of heparin per gram bead is determined by the number of layers used and also the size of the beads. The larger the bead, the less heparin per gram of bead is achieved. The surface concentration of the heparin on the solid substrate can be in the range of 1-10 µg/cm².One preferred amount is 2.0 ± 0.5 mg heparin/g bead per the MBTH method.

The molecular weight of heparin used in the claimed methods can vary. For example, native heparin has an average molecular weight of 22 kDa. Nitric acid degraded heparin has a molecular weight of 8 kDa.

Substrates useful in the present invention can also be prepared by the methods described in published U.S. Patent Application No. 2009/0136586 A1.

### Device for Use in the Methods of the Invention

Another aspect of the present invention provides use of a device comprising the heparin modified solid substrate, the heparin having a binding affinity for a cytokine or pathogen, for extracorporeal removal of the cytokine or pathogen from mammalian blood.

A device as referred to in the use and method according to the invention may comprise a conventional device for extracorporeal treatment of blood and serum from patients, e.g. suffering from renal failure.

Local blood flow patterns in blood contacting medical devices for extracorporeal circulation are known to influence clot formation via shear activation and aggregation of platelets in stagnant zones. Consequently, a device as used in the various aspects of the invention should be designed in a fashion that does not create these problems.

A device as used in some embodiments of the invention may for example have the following properties:
A blood flow in the range of 150-2000 ml/min.
Low flow resistance.
Large surface area of substrate having carbohydrates immobilized
   thereto, e.g. about 1-40 m².
Stable coating (no leakage of carbohydrate to the blood in contact therewith).
Proper haemodynamic properties in the device (no stagnant zones).
Optimal biocompatibility.

A non-limiting example of such a device, which can be used in a use or a method according to the present invention, is a pediatric haemoflow dialyzer such as the extracorporeal blood filtration device for removing cytokine molecules to be compatible with high flow rates from Exthera Medical. Other models or types of devices for extracorporeal treatment of blood or serum may also be used, such as the Prisma M10 haemofilter/dialyzer from Gambro AB, Sweden.

High-flow conditions can be defined as blood flow above the diffusion limit.

### 4. Pathogens

Further described is a method of treating a disease by removing pathogens and/or toxins from mammalian blood by contacting mammalian blood with the solid substrate disclosed in the method above. Examples of pathogens that can be removed from the blood using heparinized substrate according to the invention include: Bacteria - *Bacillus anthracis, Pseudomonas aeruginosa and Staphylococcus aureus.*

As noted above, one example of a disease to be treated is anthrax. In most cases, early treatment can cure anthrax. The cutaneous (skin) form of anthrax can be treated with common antibiotics such as penicillin, tetracycline, erythromycin and ciprofloxacin (Cipro). The pulmonary form of anthrax is a medical emergency. Early and continuous intravenous therapy with antibiotics may be lifesaving. In a bioterrorism attack, individuals exposed to anthrax will be given antibiotics before they become sick. A vaccine exists but is not yet available to the general public. There are three forms of disease caused by anthrax: cutaneous (skin) anthrax, inhalation anthrax and gastrointestinal (bowel) anthrax. Inhalation anthrax is a very serious disease, and unfortunately, most affected individuals will die even if they get appropriate antibiotics. Antibiotics are effective in killing the bacteria, but they do not destroy the deadly toxins that have already been released by the anthrax bacteria.

The methods described herein can be employed either before or after other conventional treatments, such as administration of antibiotics.

### 5. Combining the Inventions with Additional filtration/separation steps

In a further aspect the methods described above can be combined with other methods to filter or treat mammalian blood. For example, a cartridge that is based on convection kinetics can then be used in series with conventional extracorporeal circuits such as CPB, hemodialysis, and oxygenation.

### 6. Examples

The various aspects of the invention are further described in the following examples.

### 6.1. Example 1 - Preparation of heparin column

Polyethylene (PE) beads, with an average diameter of 0.3 mm (lot no. 180153), are supplied by the Polymer Technology Group (Berkeley, USA) and the columns (Mobicol, 1 mL) are obtained from MoBiTec (Germany). Heparin and polyethyleneimine (PEI) are purchased from Scientific Protein Laboratories (Waunakee, Wisconsin, USA) and BASF (Ludwigshafen, Germany) respectively. All chemicals used are of analytical grade or better. Immobilization of heparin onto the beads are performed as described by Larm et al. (Larm O, Larsson R, Olsson P. A new non-thrombogenic surface prepared by selective covalent binding of heparin via a modified reducing terminal residue. Biomater Med Devices Artif Organs 1983; 11: 161-173). The polymeric surface is heparinized using the general procedure described below.

The polymeric surface is etched with a oxidizing agent (potassium permanganate, ammoniumperoxidisulfate) in order to introduce hydrophilic characteristics together with some reactive functional groups (-SO₃H, -OH,-C=O, -C=C-). The surface can also be etched with plasma or corona. For example, the PE- beads are etched with an oxidizing agent (potassium permanganate in sulphuric acid). These hydrophilized beads, *inter alia* containing OH-groups and double bonds, are later used as controls.

Reactive amino functions are introduced by treatment with a polyamine, polyethylenimine (PEI) or chitosan. For some purposes the polyamines may be stabilized on the surface by cross linking with bifunctional reagents, such as crotonaldehyde or glutaraldehyde.

The coating is further stabilized by ionic cross linking with a sulfated polysaccharide (dextran sulfate or heparin). If necessary these steps are repeated and a sandwich structure is built up. Careful rinsing (water, suitable buffers) should be performed between each step. After a last addition of PEI or chitosan, end-point attachment (EPA) to the aminated surface of native heparin is done by reductive amination, utilizing the aldehyde function in the reducing terminal residue in native heparin.

A more reactive aldehyde function in the reducing terminal residue can be achieved by partial, nitrous degradation of heparin. This shortens the reaction time, but the immobilized heparin will have a lower molecular weight. The coupling is performed in aqueous solution, by reductive amination (cyanoborohydride, CNBH₃⁻).

In this alternate method, the aminated media is suspended in acetate buffer (800 ml, 0.1 M, pH 4.0) and 4.0 g nitrous acid degraded heparin (heparin from Pharmacia, Sweden) was added. After shaking for 0.5 h, NaBH₃CN (0.4 g) was added. The reaction mixture was shaken for 24 h and then processed as above, yielding heparinized media.

1-10 µg/cm² of heparin can be coupled to all hydrophilic surfaces like glass, cellulose, chitin etc, and more or less all hydrophobic polymers like polyvinyl chloride, polyethylene, polycarbonate, polystyrene, PTFE etc.

The resulting PE-beads, with covalently end-point attached heparin, are sterilized with ethylenoxide (ETO) and rinsed with 0.9% sodium chloride and ultra pure water. The amount heparin was determined to be 2.0 mg heparin/g bead with the MBTH method. (Larm O, Larsson R, Olsson P. A new non-thrombogenic surface prepared by selective covalent binding of heparin via a modified reducing terminal residue. Biomater Med Devices Artif Organs 1983; 11: 161-173 and Riesenfeld J, Roden L. Quantitative analysis of N-sulfated, N-acetylated, and unsubstituted glucosamine amino groups in heparin and related polysaccharides. Anal Biochem 1990; 188: 383-389).

The polyethylene beads have a mean diameter of 0.3 mm and are heparinized with a technology that guaranteed that the heparin molecules are covalently end point attached to the surface, thereby making the carbohydrate chains more accessible for proteins with affinity for heparin/heparin sulphate. The mean molecular weight of the immobilized heparin is about 8 kDa, while 2 mg (equal to approximately 360 IU) is coupled to each gram of beads. The integrity of this surface is verified by the expected removal of 75% of antithrombin (AT) concentrations from the blood passed over heparinized, but not non-heparinized, beads.

### 6.2. Example 2 - Removal of Toxins

Arterial blood is drawn from the hemodialyzers of patients. The blood is collected in EDTA vacuum tubes and immediately 1 mL is applied to the previously prepared columns and passed through using a roller-pump at, for example, one of 1, 5 and 10 mL/min. Blood that has passed through the columns is immediately collected at the other end and cold-centrifuged (4500 G). The supernatants are subsequently collected and frozen at -80° C for later analysis.

Briefly, passage through the heparinised beads results in a significantly bigger decrease in blood toxins as compared to non-heparinized beads.

### 6.3. Example 3: In vitro removal of B. anthracis PA

**Bacterial Supernatants:** Overnight cultures of *B*. *anthracis* 7702 or 9131 were cultured in Luria Broth (LB) at 37°C while shaking at 250 RPM. 20 mLs of LB with 0.8% sodium bicarbonate (NaHCO₃) at pH 8 (pH with 1 M HEPES) was inoculated with 1 mL overnight 7702 culture and grown until late exponential phase (approximately 7 hours) while shaking at 250 RPM at 37°C. Cultures were centrifuged for 5 minutes at 3500 RPM to remove bacterial cells and debris. Supernatants were collected and passed through a 0.2 µm filter and used immediately or stored at -20°C.

**Preparation of Beads:** 1 g heparin or control beads were added to syringes with a filter placed in the bottom and on top of the beads. Prior to experiments, beads were prepped by the addition of 2 mLs Tris-buffered Saline (TBS). Where Fetal Bovine Serum (FBS) was used, the beads were prepped by the addition of 2 mLs TBS followed by 2 mLs FBS, which was passaged over the beads 5 times. When drops of TBS or FBS where no longer released from the syringes, bacterial supernatants were passaged.

**Supernatant Passage:** 2 mLs of bacterial supernatant was applied to the beads. After passage, the supernatant was collected and passaged through an additional 4 times. When drops of supernatant were no longer released from the syringes, the supernatant was collected at kept at -20°C.

**ELISA:** 100 µL supernatant was added to each well of an Immulon 4HBX high binding microtiter plate and incubated at room temperature for 2 hours on a rocker. The supernatant was removed and wells were washed 3 times with TBS containing 0.05% Tween (TBST). Well were blocked with 2% bovine serum albumin for 1 hour at room temperature on a rocker. Wells were washed again. Wells were incubated with goat anti-PA (List Biological Laboratories) (1:2000 dilution in TBS) for 1 hour at room temperature on a rocker. Wells were washed as described. Wells were incubated with rabbit anti-goat-HRP (Invitrogen) (1:2000 dilution in TBS) for 1 hour at room temperature on a rocker. Wells were washed as described. Wells were developed by the addition of SigmaFast-OPD (Sigma) in dH₂O for approximately 30 minutes at room temperature. Absorbance was read at 450 nm.

**Western Blot:** Bacterial supernatants were obtained as described and 2.5 µL or 5 µL volumes were added to a PVDF membrane (BioTrace) that was prepped in methanol, followed by TBS for 3 minutes. After 5 minutes, the membrane was blocked with 2% non-fat milk in TBST for 30 minutes at room temperature. The membrane was washed 3 times with TBST and incubated with 1:3000 dilution of goat anti-PA for 45 minutes at room temperature. The membrane was washed 3 times and incubated with 1:3000 rabbit anti-goat-alkaline phosphatase for 45 minutes at room temperature. The membrane was developed with 1-step NBT/NCIP (Piercenet).

### Results

It was first verified that toxin was produced under our culturing conditions using a Western/dot blot assay. Protective antigen (PA) was detected in 7702 cultures with and without atmospheric conditions of 5% CO₂ at 37°C in both overnight and 8 hour cultures. FBS was used as a control for background antibody detection.

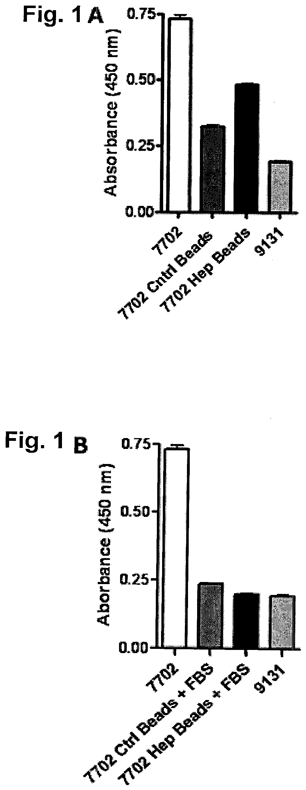

7702 and 9131 supernatants were passed through control and heparinized beads. We found approximately 75% reduction with control and heparinized beads (Figure 1, B). Compilations of replicates, to date, indicate a 43.3% reduction without pre-soak and a 75% reduction with a pre-soak after application to heparinized beads. Reduction of 75% brings the measurement of PA near background levels of 9131 indicating that there may be a 100% reduction. Reductions in PA were recovered after one passage of supernatants over the beads. Multiple passages had no effect on the reduction of PA.

### 6.4. Example 4: Demonstration of Cell Protection by Heparinized Beads.

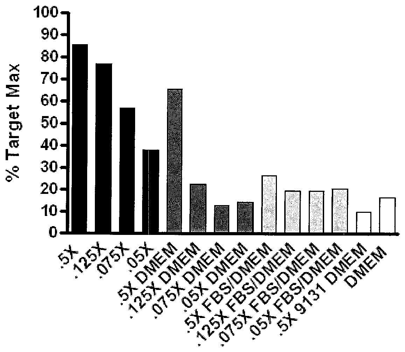

In this study, PA supernatant and bead preparation were performed as outlined in example 3. Supernatents 7702 and 9131 were concentrated 7702 and 9131 to 10x. The supernatant was then diluted to desire concentration in DMEM cell culture media (-phenol red). Macrophages were cultured, counted, and resuspended to 1x10^6 cells/ml. 1x10^5 cells were added to each well in 500 µl DMEM+FBS and incubated overnight at 37C and 5% CO2. 0.05 g beads were added to each well. DMEM +/-FBS or FBS were then passed through the transwell in 100 µl increments (total 300 µl). The media was then removed from the culture wells and the beads were washed once with DMEM. 500 µl diluted supernatants were then added to each transwell. The wells were then cultured for 20 hrs at 37C/5% CO2. 50 ml were sampled from each transwell and LDH levels were measured (cytotoxicity). Figure 2 shows the results. A significant reduction in Macrophage cell death was measured regardless of supernatant dilution. For beads that were treated with FBS and DMEM, cell death was reduced to background levels.

### 6.5. Example 5: Removal of strain USA300 methicilin resistant S. Aureus α-toxin.

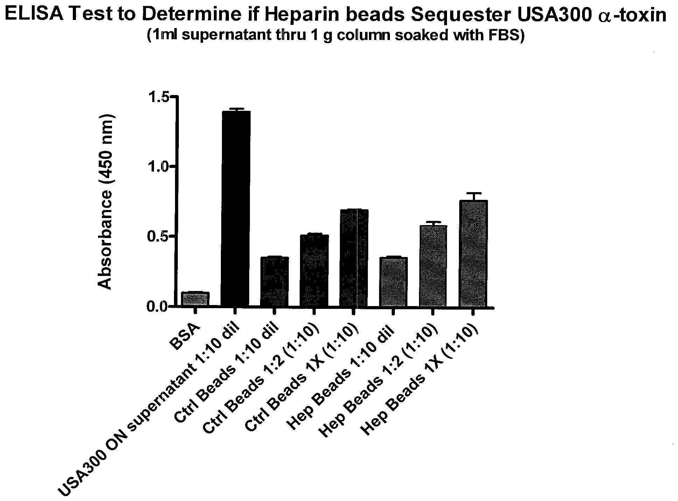

Supernatants with USA300 MRSA α-toxin was prepared using strain USA300 following the procedure outlined in Example 3. The heparinized beads were also prepared following the procedure outlined in Example 3. Figure 3 shows the results. The concentration of α-toxin was significantly reduced regardless of supernatant dilution as measured by ELISA-type assay.

## Claims

1. A method for removal from mammalian blood of a toxin from a pathogen wherein said pathogen is a member selected from the group consisting of *Bacillus anthracis, Pseudomonas aeruginosa,* and *Staphylococcus aureus,* wherein the toxin from *Bacillus anthracis* is a member selected form the group of the anthrax lethal toxin, anthrax protective antigen, anthrax edema factor, anthrax lethal factor, anthrax polyglutamic acid capsule, anthralysin O, anthralysin B; wherein the toxin from *Pseudomonas aeruginosa* is Las A; and wherein the toxin from *Staphylococcus aureus* is a member selected from the group consisting of S. aureus α-toxin, and S. aureus β-toxin, said method comprising:
a. bringing a sample of blood in contact with a carbohydrate immobilized on a solid substrate, said carbohydrate having binding affinity for said toxin, under conditions allowing the binding of said substrate to said toxin in said sample of blood;
b. separating the sample from said substrate whereby said toxin is at least partially retained or said substrate and the removed sample has a reduced amount of toxin.

2. The method according to claim 1, wherein said pathogen is *Bacillus anthracis.*

3. The method according to claim 1, wherein said substrate has affinity to toxins that are capable of binding to heparin sulphate segments on syndecans.

4. The method according to claim 1, wherein said substrate has affinity to the protective antigen in anthrax toxin.

5. The method according to any one of claims 1-4, wherein said solid substrate is comprised of at least one member selected from the group consisting of glass, cellulose, cellulose acetate, chitin, chitosan, crosslinked dextran, crosslinked agarose, polyurethane, polymethylmethacrylate, polyethylene or co-polymers of ethylene and other monomers, polyethylene imine, polypropylene, polysulfone, polyacrylonitrile, silicone and polyisobutylene.

6. The method according to any one of claims 1-5, wherein said carbohydrate is heparin.

7. The method according to any one of the preceding claims, said method comprising:
causing a sample of blood or serum to flow over and past a high-surface-area solid substrate at a flow rate of ≥50 ml/min wherein the surface of said solid substrate comprises heparin with a binding affinity for the toxin, wherein said substrate is sufficiently nonporous such that adsorbents in the blood do not need to pass through pores in said substrate prior to adsorption, and wherein the size of the interstitial channel spaces between individual portions of said substrate and the amount of interstitial substrate surface area is such that when said flowing blood or serum in contact with said substrate at a flow rate of ≥50 ml/min, said toxin binds to said heparin to separate from said blood or serum and the transport of said blood or serum and adsorbents contained in it past said substrate is by means of convection transport more than Brownian diffusion transport.

8. The method according to claim 7, wherein said substrate comprises a packed column of non-porous rigid beads or particles, a column packed with a rigid reticulated foam, a column packed with a rigid monolith bed of sintered beads with internal flow channels, a column packed with woven or non woven rigid fabric, a column packed with a rigid yarn or an optionally hollow monofilament, a spiral wound cartridge, or a combination of at least two members selected from the group consisting of beads, rigid reticulated foam, sintered beads, fabric, yarn and monofilament.

9. The method according to claim 7, wherein the solid substrate comprises polyethylene beads coated with one or more polysaccharides.

10. The method according to claim 9, wherein at least one of said polysaccharides is selected from the group consisting of heparin, hyaluronic acid, salicylic acid, and chitosan.

11. The method according to any one of the preceding claims, said method comprising:
causing a sample of blood to flow in contact with rigid polyethylene beads in a container at a flow rate of ≥50 ml/min, wherein the surface of said beads comprises heparin with a binding affinity for the toxin, wherein said beads are sufficiently rigid such that blood does not pass through pores in said beads, and wherein the size of the interstitial channel spaces between individual ones of said beads and the amount of interstitial surface area of said beads is such that when said sample of blood is in flow contact with said substrate at a flow rate of ≥50 ml/min, said toxin binds to said heparin to separate from said blood and the flow transport of said blood past said substrate is by means of convection transport more than Brownian diffusion transport method.

12. The method of any one of claims 7-11, wherein the flow rate of blood or serum ranges from about 150 and 2000 mL/minute.

13. The method of any one of claims 2-12, wherein the bead comprises a polymer of polyethylene.

14. The method of any one of claim 2-13, wherein said beads have a diameter ranging from 100 to 450 microns, such as an average diameter of 0.3 mm.

15. The method of any one of claims 2-14, wherein the beads are coated with 0.5 - 10 mg heparin per gram of bead, such as coated with 2 ± 0.5 mg heparin per gram of bead.

16. The method of any one of claims 1-15, wherein the heparin has a mean molecular weight of about 8 kDa.

17. The method of any one of claims 1-16, wherein the heparin is attached to the bead by covalent end-point attachment.

18. The method according to any one of claims 1-17, wherein said method for removal is suitable for being conducted in combination with at least one other extracorporeal treatment of said blood.

19. The method according to claim 18, wherein said at least one other extracorporeal treatment comprises at least one member selected from the group consisting of cardiopulmonary bypass (CPB), hemodialysis, and oxygenation.

## Patentansprüche

1. Verfahren zur Entfernung eines Toxins von einem Krankheitserreger aus Säugetierblut, wobei der Krankheitserreger ein Element ausgewählt aus der Gruppe bestehend aus *Bacillus anthracis, Pseudomonas aeruginosa* und *Staphylococcus aureus* ist, wobei das Toxin von *Bacillus anthracis* ein Element ausgewählt aus der Gruppe des tödlichen Anthraxtoxins, einem schützenden Anthraxantigen, einem Anthrax-Ödemfaktor, einem tödlichen Anthraxfaktor, der Anthrax-Polyglutaminsäurekapsel, Anthralysin O, Anthralysin B, ist; wobei das Toxin von *Pseudomonas aeruginosa* Las A ist; und wobei das Toxin von *Staphylococcus aureus* ein Element ausgewählt aus der Gruppe bestehend aus dem α-Toxin von *S*. *aureus* und dem β-Toxin von *S*. *aureus* ist, wobei das Verfahren Folgendes umfasst:
a. Inkontaktbringen einer Blutprobe mit einem auf einem festen Substrat immobilisierten Kohlenhydrat, wobei das Kohlenhydrat eine Bindungsaffinität für das Toxin aufweist, unter Bedingungen, welche die Bindung des Substrats an das Toxin in der Blutprobe ermöglichen;
b. Trennen der Probe von dem Substrat, wodurch das Toxin mindestens teilweise zurückgehalten wird oder das Substrat und die entfernte Probe eine verringerte Menge an Toxin aufweisen.

2. Verfahren nach Anspruch 1, wobei das Pathogen *Bacillus anthracis* ist.

3. Verfahren nach Anspruch 1, wobei das Substrat eine Affinität zu Toxinen aufweist, die in der Lage sind, an Heparinsulfatsegmente auf Syndecanen zu binden.

4. Verfahren nach Anspruch 1, wobei das Substrat Affinität zu dem schützenden Antigen im Anthraxtoxin aufweist.

5. Verfahren nach einem der Ansprüche 1 bis 4, wobei das feste Substrat mindestens ein Element ausgewählt aus der Gruppe bestehend aus Glas, Cellulose, Celluloseacetat, Chitin, Chitosan, vernetztem Dextran, vernetzter Agarose, Polyurethan, Polymethylmethacrylat. Polyethylen oder Copolymeren von Ethylen und anderen Monomeren, Polyethylenimin, Polypropylen, Polysulfon, Polyacrylnitril, Silikon und Polyisobutylen umfasst.

6. Verfahren nach einem der Ansprüche 1 bis 5, wobei das Kohlenhydrat Heparin ist.

7. Verfahren nach einem der vorhergehenden Ansprüche, wobei das Verfahren Folgendes umfasst:
Veranlassen einer Blut- oder Serumprobe zum Hinwegfließen über ein festes Substrat mit großer Oberfläche mit einer Flussrate von ≥ 50 ml/min, wobei die Fläche des festen Substrats Heparin mit einer Bindungsaffinität für das Toxin umfasst, wobei das Substrat ausreichend unporös ist, damit adsorbierende Stoffe in dem Blut vor der Adsorption nicht die Poren in dem Substrat passieren müssen, und wobei die Größe der Zwischenkanalräume zwischen einzelnen Abschnitten des Substrats und die Menge an Zwischensubstratoberfläche derart ist, dass das Toxin an das Heparin bindet, wenn sich das fließende Blut oder Serum bei einer Flussrate von ≥ 50 ml/min in Kontakt mit dem Substrat befindet, um sich von dem Blut oder Serum zu trennen, und der Transport des Bluts oder Serums und der darin enthaltenen adsorbierenden Stoffe über das Substrat hinweg eher durch Konvektionstransport als durch Brownschen Diffusionstransport stattfindet.

8. Verfahren nach Anspruch 7, wobei das Substrat eine gepackte Säule aus unporösen starren Perlen oder Partikeln, eine mit einem starren retikuliertem Schaumstoff gepackte Säule, eine mit einem starren Monolithbett aus gesinterten Perlen mit inneren Strömungskanälen gepackte Säule, eine mit gewebtem oder nicht gewebtem starrem Gewebe gepackte Säule, eine mit einem starren Garn oder einem gegebenenfalls hohlen Monofilament gepackte Säule, eine spiralförmig gewickelte Kartusche oder eine Kombination von mindestens zwei Elementen ausgewählt aus der Gruppe bestehend aus Perlen, starrem retikuliertem Schaum, gesinterten Perlen, Gewebe, Garn und Monofilament umfasst.

9. Verfahren nach Anspruch 7, wobei das feste Substrat Polyethylenperlen umfasst, die mit einem oder mehreren Polysacchariden überzogen sind.

10. Verfahren nach Anspruch 9, wobei mindestens eines der Polysaccharide ausgewählt ist aus der Gruppe bestehend aus Heparin, Hyaluronsäure, Salicylsäure und Chitosan.

11. Verfahren nach einem der vorhergehenden Ansprüche, wobei das Verfahren Folgendes umfasst:
Veranlassen einer Blut- oder Serumprobe zum Fließen in Kontakt mit starren Polyethylenperlen in einem Behälter bei einer Flussrate von ≥ 50 ml/min, wobei die Oberfläche der Perlen Heparin mit einer Bindungsaffinität für das Toxin umfasst, wobei die Perlen ausreichend starr sind, dass Blut die Poren in den Perlen nicht passiert, und wobei die Größe der Zwischenkanalräume zwischen einzelnen der Perlen und die Menge an Zwischenoberfläche der Perlen derart ist, dass das Toxin an das Heparin bindet, wenn sich die Blutprobe bei einer Flussrate von ≥ 50 ml/min in Flusskontakt mit dem Substrat befindet, um sich von dem Blut zu trennen, und der Flusstransport des Bluts über das Substrat hinweg eher durch Konvektionstransport als durch das Brownsche Diffusionstransportverfahren stattfindet.

12. Verfahren nach einem der Ansprüche 7 bis 11, wobei die Flussrate von Blut oder Serum im Bereich von etwa 150 bis 2000 ml/min liegt.

13. Verfahren nach einem der Ansprüche 2 bis 12, wobei die Perle ein Polymer aus Polyethylen umfasst.

14. Verfahren nach einem der Ansprüche 2 bis 13, wobei die Perlen einen Durchmesser im Bereich von 100 bis 450 Mikrometern haben, beispielsweise einen durchschnittlichen Durchmesser von 0,3 mm.

15. Verfahren nach einem der Ansprüche 2 bis 14, wobei die Perlen mit 0,5 bis 10 mg Heparin pro Gramm Perle überzogen sind, beispielsweise mit 2 ± 0,5 mg Heparin pro Gramm Perle beschichtet sind.

16. Verfahren nach einem der Ansprüche 1 bis 15, wobei das Heparin ein mittleres Molekulargewicht von etwa 8 kDa hat.

17. Verfahren nach einem der Ansprüche 1 bis 16, wobei das Heparin durch kovalente Endpunktbefestigung an der Perle befestigt ist.

18. Verfahren nach einem der Ansprüche 1 bis 17, wobei das Verfahren zur Entfernung geeignet ist, um in Kombination mit mindestens einer anderen extrakorporalen Behandlung des Blutes durchgeführt zu werden.

19. Verfahren nach Anspruch 18, wobei die mindestens eine andere extrakorporale Behandlung mindestens ein Element ausgewählt aus der Gruppe bestehend aus kardiopulmonalem Bypass (CPB), Hämodialyse und Oxygenierung umfasst.

## Revendications

1. Méthode consistant à éliminer, de sang de mammifère, une toxine provenant d'un pathogène, dans laquelle ledit pathogène est un élément sélectionné dans le groupe constitué par *Bacillus anthracis, Pseudomonas aeruginosa* et *Staphylococcus aureus,* dans laquelle la toxine provenant de *Bacillus anthracis* est un élément sélectionné dans le groupe de la toxine létale de l'anthrax, l'antigène protecteur de l'anthrax, le facteur oedématogène de l'anthrax, le facteur létal de l'anthrax, la capsule d'acide polyglutamique de l'anthrax, l'anthralysine O, l'anthralysine B ; dans laquelle la toxine provenant de *Pseudomonas aeruginosa* est Las A ; et dans laquelle la toxine provenant de *Staphylococcus aureus* est un élément choisi dans le groupe constitué par la toxine α de S. aureus et la toxine β de S. aureus, ladite méthode comprenant :
a. la mise en contact d'un échantillon de sang avec un glucide immobilisé sur un substrat solide, ledit glucide ayant une affinité de liaison pour ladite toxine, dans des conditions permettant la liaison dudit substrat à ladite toxine dans ledit échantillon de sang ;
b. la séparation de l'échantillon dudit substrat selon laquelle ladite toxine est au moins partiellement retenue sur ledit substrat et l'échantillon prélevé a une teneur réduite en toxine.

2. Méthode selon la revendication 1, dans laquelle ledit pathogène est *Bacillus anthracis.*

3. Méthode selon la revendication 1, dans laquelle ledit substrat a une affinité vis-à-vis des toxines qui sont capables de se lier à des segments de sulfate d'héparine sur des syndécanes.

4. Méthode selon la revendication 1, dans laquelle ledit substrat a une affinité vis-à-vis de l'antigène protecteur dans la toxine de l'anthrax.

5. Méthode selon l'une quelconque des revendications 1 à 4, dans laquelle ledit substrat solide se compose d'au moins un élément sélectionné dans le groupe constitué par le verre, la cellulose, l'acétate de cellulose, la chitine, le chitosane, le dextrane réticulé, l'agarose réticulé, le polyuréthane, le polyméthacrylate de méthyle, le polyéthylène ou des copolymères d'éthylène et d'autres monomères, la polyéthylène imine, le polypropylène, la polysulfone, le polyacrylonitrile, le silicone et le polyisobutylène.

6. Méthode selon l'une quelconque des revendications 1 à 5, dans laquelle ledit glucide est l'héparine.

7. Méthode selon l'une quelconque des revendications précédentes, ladite méthode comprenant :
le fait de faire couler un échantillon de sang ou de sérum sur et à travers un substrat solide de grande superficie à un débit ≥ 50 ml/min, la surface dudit substrat solide comprenant de l'héparine ayant une affinité de liaison pour la toxine, ledit substrat étant suffisamment non poreux pour que les adsorbants dans le sang n'aient pas besoin de passer à travers les pores dudit substrat avant l'adsorption, et la taille des espaces des canaux interstitiels entres les parties individuelles dudit substrat et la quantité de superficie de substrat interstitielle est telle que, lorsque ledit écoulement de sang ou de sérum en contact avec ledit substrat à un débit ≥ 50 ml/min, ladite toxine se lie à ladite héparine pour être séparée dudit sang ou sérum et le transport dudit sang ou sérum et des adsorbants contenus dans celui-ci passant à travers ledit substrat se fait plus par un moyen de transport par convection que par un transport par diffusion brownienne.

8. Méthode selon la revendication 7, dans laquelle ledit substrat comprend une colonne remplie de billes ou de particules rigides non poreuses, une colonne remplie d'une mousse réticulée rigide, une colonne remplie d'un lit monolithique rigide de billes frittées avec des canaux d'écoulement internes, une colonne remplie avec un tissu rigide tissé ou non tissé, une colonne remplie avec un fil rigide ou un monofilament éventuellement creux, une cartouche enroulée en spirale, ou une combinaison d'au moins deux éléments sélectionnés dans le groupe constitué par des billes, de la mousse réticulée rigide, des billes frittées, un tissu, un fil et un monofilament.

9. Méthode selon la revendication 7, dans laquelle le substrat solide comprend des billes de polyéthylène enrobées d'un ou plusieurs polysaccharides.

10. Méthode selon la revendication 9, dans laquelle au moins un desdits polysaccharides est sélectionné dans le groupe constitué par l'héparine, l'acide hyaluronique, l'acide salicylique et le chitosane.

11. Méthode selon l'une quelconque des revendications précédentes, ladite méthode comprenant :
le fait de faire couler un échantillon de sang en contact avec des billes de polyéthylène rigides dans un contenant à un débit ≥ 50 ml/min, la surface desdites billes comprenant de l'héparine ayant une affinité de liaison pour la toxine, lesdites billes étant suffisamment rigides pour que le sang ne passe pas à travers les pores desdites billes, et la taille des espaces des canaux interstitiels entre les billes individuelles desdites billes et la quantité de superficie interstitielle desdites billes est telle que lorsque ledit échantillon de sang est en contact d'écoulement avec ledit substrat à un débit ≥ 50 ml/min, ladite toxine se lie à ladite héparine pour être séparée dudit sang et le transport par écoulement dudit sang passant à travers ledit substrat se fait plus par un moyen de transport par convection que par une méthode de transport par diffusion brownienne.

12. Méthode selon l'une quelconque des revendications 7 à 11, dans laquelle le débit de sang ou de sérum est compris dans la plage allant d'environ 150 à 2 000 ml/minute.

13. Méthode selon l'une quelconque des revendications 2 à 12, dans laquelle la bille comprend un polymère de polyéthylène.

14. Méthode selon l'une quelconque des revendications 2 à 13, dans laquelle lesdites billes ont un diamètre compris dans la plage allant de 100 à 450 micromètres, comme un diamètre moyen de 0,3 mm.

15. Méthode selon l'une quelconque des revendications 2 à 14, dans laquelle les billes sont enrobées avec de 0,5 à 10 mg d'héparine par gramme de bille, comme enrobées avec 2 ± 0,5 mg d'héparine par gramme de bille.

16. Méthode selon l'une quelconque des revendications 1 à 15, dans laquelle l'héparine a un poids moléculaire moyen d'environ 8 kDa.

17. Méthode selon l'une quelconque des revendications 1 à 16, dans laquelle l'héparine est attachée à la bille par un attachement d'extrémité covalent.

18. Méthode selon l'une quelconque des revendications 1 à 17, ladite méthode d'élimination étant appropriée pour être réalisée en combinaison avec au moins un autre traitement extracorporel dudit sang.

19. Méthode selon la revendication 18, dans laquelle ledit au moins un autre traitement extracorporel comprend au moins un élément sélectionné dans le groupe constitué par la dérivation cardio-pulmonaire (CPB), l'hémodialyse et l'oxygénation.
